# EUROPEAN PATENT APPLICATION

(11) **EP 4 790 141 A1**
(43) Date of publication of application: **12.08.2026**
(21) Application number: 24876393.0
(22) Date of filing: 25.09.2024
(51) Int. Cl.: C12N 15/113, C12N 5/10, C12N 15/85, C12P 21/00

(54) **TRUNCATED INSULATOR AND USE THEREOF IN TRANSIENT EXPRESSION OF RECOMBINANT PROTEIN**

(30) Priority: 08.10.2023 CN 202311295717
(71) Applicant: Shanghai Qilu Pharmaceutical Research and Development Centre Ltd., Shanghai 201203 (CN)
(72) Inventor: CHEN, Xi, Shanghai 201203 (CN); FANG, Yan, Shanghai 201203 (CN); SHI, Lani, Shanghai 201203 (CN); JIN, Congcong, Shanghai 201203 (CN); CHEN, Jie, Shanghai 201203 (CN); ZHANG, Kang, Shanghai 201203 (CN)
(74) Representative: dompatent
(86) International application number: PCT/CN2024/121044
(87) International publication number: WO 2025/077575

(57) **Abstract**

A truncated insulator, an expression system comprising the truncated insulator and a transposon (PiggyBac) element, and a method for transient expression and production of a recombinant protein by using the expression system.

## Description

The present application claims priority to Patent Application No. 202311295717.9 entitled "TRUNCATED INSULATOR AND USE THEREOF IN TRANSIENT EXPRESSION OF RECOMBINANT PROTEIN" and filed with China National Intellectual Property Administration on October 8, 2023, which is incorporated into the present disclosure by reference in its entirety.

### TECHNICAL FIELD

The present disclosure relates to the field of biotechnology, and in particular to a truncated insulator, an expression system comprising same and a transposon (PiggyBac) element, and a method for producing a protein by transient expression using same.

### BACKGROUND

So far, as many as hundreds of antibody drugs have been approved globally. The antibody market has reached a scale of hundreds of billions of dollars. Antibody drugs in various therapeutic fields are increasingly benefiting patients. Antibody drugs for treatment need to concurrently meet multiple strict criteria of, e.g., efficacy, stability, immunogenicity, pharmacodynamics, pharmacokinetics, ease of expression and purification, and the like, and usually require several rounds of engineering design and process optimization. Each round of design and optimization involves quantities of proteins for necessary characterization, therefore creating a need for the rapid production of quantities of purified proteins of high quality.

It has been reported that insulators play a crucial role in regulating the spatiotemporal specific expression of eukaryotic genes. They can protect the target gene from the influence of surrounding regulatory factors, so as to prevent incorrect gene activation or silencing, i.e., enhancer blocking and heterochromatin barrier functions, which can effectively inhibit the "position effect". HS4 is a DNase I hypersensitive site (HS) having an insulator function located upstream at the 5' end of the chicken β globulin gene locus, i.e., cHS4, with a length of about 1.2 kp, which, in some studies, has been demonstrated to possess both of the above two functions. However, existing studies have shown that the insertion of an intact sequence results in an oversized vector and reduced vector transfection efficiency, thereby affecting the expression amount of the protein. Therefore, research on the truncation of cHS4 is needed. Studies have shown that the function of cHS4 depends on the 250-bp core region at the 5' end, but the function of the core region is insufficient and cannot well improve the expression. The 400-bp sequence at the 3' end also plays an important role, and the effect of cHS4-650 (the 250-bp core region and the 400-bp sequence at the 3' end) is better than that of cHS4-250. However, the 650-bp insulator still has the problems of oversizing and reduced transfection efficiency. Therefore, the technology for further optimizing the length of the insulator sequence on the basis of the 650-bp length and improving the transfection efficiency is demanded. So far, however, most of the current studies on the truncation of the cHS4 insulator are based on the strategy of combining different segments that have been studied, mainly through the combination of the 250-bp core region with other different segments, without further optimizing the length of the insulator or verifying the function of the truncated insulator. Moreover, such studies have not actually been applied to the construction of stably transfected cell lines for antibody drug production, and are still far from industrial application.

Transient expression is a method for the rapid production of recombinant proteins on the milligram to gram scale. Unlike stable expression, the gene expressing the target protein does not need to be stably integrated into the genome of the host cell but is present in the transfected cells in the form of a plasmid DNA, nor does the plasmid DNA need to be maintained by a screening pressure. Compared with stable expression, transient expression is primarily characterized by: (1) enabling rapid expression of the protein, which generally takes only 1-3 weeks; (2) the capability of applying to various different recombinant proteins, including proteins toxic to host cells. Due to these characteristics, transient expression is an ideal method for rapid production of proteins. However, generally the yield of the transiently expressed protein is relatively low, typically within the range of 60-80 mg/L.

In order to increase the yield of transient expression, researchers have developed various different methods, such as increasing the host cell density, co-transfection with an expression-promoting gene, addition of an enhancing reagent, and the like, which involve additional procedures and meanwhile increase the cost. The PiggyBac transposon can promote gene integration and protein expression, but conventional PiggyBac transposon expression systems usually involve procedures of stressed screening and cell recovery that are time-consuming. Meanwhile, the limited protein expression amount still cannot meet the requirement of rapid protein preparation. Therefore, a transient expression system with a simple process, low industrial costs, and high yield is needed.

### SUMMARY

In one aspect, the present disclosure provides an insulator for polypeptide or protein expression in a eukaryotic cell (see WO2024146587A1). The insulator comprises the nucleic acid sequence shown in SEQ ID NO: 1, SEQ ID NO: 2, SEQ ID NO: 3, or SEQ ID NO: 4, or comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to any one of the nucleic acid sequences shown in SEQ ID NO: 1, SEQ ID NO: 2, SEQ ID NO: 3, and SEQ ID NO: 4; preferably, the insulator is the nucleic acid sequence shown in SEQ ID NO: 1, SEQ ID NO: 2, SEQ ID NO: 3, or SEQ ID NO: 4, or is a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to any one of the nucleic acid sequences shown in SEQ ID NO: 1, SEQ ID NO: 2, SEQ ID NO: 3, and SEQ ID NO: 4.

The present disclosure provides an expression vector comprising the insulator of the present disclosure. Preferably, the expression vector is a PiggyBac transposon transgenic vector; preferably, the expression vector does not comprise a marker gene for screening, or preferably, the expression vector comprises a marker gene for screening.

The present disclosure further provides an expression system comprising the insulator of the present disclosure; preferably, the expression system is an expression system of a eukaryotic cell, more preferably, the expression system is a PiggyBac transposon expression system.

Preferably, the PiggyBac transposon expression system comprises a PiggyBac transposon transgenic vector; preferably, the PiggyBac transposon expression system further comprises a PiggyBac helper vector or an mRNA encoding a PB transposase, and the PiggyBac helper vector comprises a nucleic acid encoding the PB transposase; preferably, the PiggyBac helper vector is constructed from pcDNA3.4.

Preferably, the PiggyBac transposon transgenic vector comprises the insulator of the present disclosure. Preferably, the PiggyBac transposon transgenic vector comprises a 5' terminal repeat 5ITR and an inverted 3' terminal repeat 3ITR, and the insulator is arranged between the 5ITR and the 3ITR; preferably, two insulators that inverted repeats or direct repeats are sequentially arranged between the 5ITR and the 3ITR; preferably, a multiple cloning site is arranged between the insulators; preferably, the other elements of the PiggyBac transposon transgenic vector are derived from an original sequence of a P3 vector.

Preferably, the PiggyBac transposon transgenic vector does not comprise a marker gene for screening; or preferably, the PiggyBac transposon transgenic vector comprises a marker gene for screening.

In another aspect, the present disclosure provides a method for using the expression system, particularly a method for gene recombination and transient expression. The method comprises the following steps:
1) constructing an expression system comprising a gene encoding a target protein;
2) introducing the expression system in step 1) into a host cell by transfection; and
3) using a pool of the transfected cells for expression production after the transfection;
wherein the expression system comprises the insulator of the present disclosure. Preferably, the expression system is an expression system of a eukaryotic cell. Preferably, the expression system is a PiggyBac transposon expression system; preferably, the PiggyBac transposon expression system comprises a PiggyBac transposon transgenic vector; preferably, the PiggyBac transposon transgenic vector comprises a 5' terminal repeat 5ITR and an inverted 3' terminal repeat 3ITR, and the insulator is arranged between the 5ITR and the 3ITR; preferably, two insulators that are inverted repeats or direct repeats are sequentially arranged between the 5ITR and the 3ITR; preferably, a multiple cloning site is arranged between the insulators; preferably, the other elements of the PiggyBac transposon transgenic vector are derived from an original sequence of a P3 vector; preferably, the PiggyBac transposon transgenic vector does not comprise a marker gene for screening, or preferably, the PiggyBac transposon transgenic vector comprises a marker gene for screening; preferably, the PiggyBac transposon expression system further comprises a PiggyBac helper vector or an mRNA encoding a PB transposase, and the PiggyBac helper vector comprises a nucleic acid encoding a PB transposase; preferably, the host cell is a eukaryotic host cell; more preferably, the host cell is a CHO cell.

Preferably, the target protein in step 1) may be an antibody, a fusion protein, an antigen, an enzyme, or a protein or polypeptide of other types.

Preferably, in step 1), a PB transposase-encoding mRNA is obtained by *in vitro* transcription, capping, and tailing modifications using a DNA sequence encoding the PB transposase as a template by means of the *in vitro* transcription method.

Preferably, in step 1), an endotoxin-free plasmid extraction kit is used for vector construction.

Preferably, in step 2), the transfection is electrotransfection, chemical transfection, or other transfection methods.

Preferably, in step 3), the duration after the transfection is 1 day, 2 days, 3 days, or other period after the transfection.

Preferably, in step 3), no stressed screening is required after the transfection or before the production. Preferably, in step 3), the expression production is transient expression production.

Preferably, in step 3), the expression production is a fed-batch culture.

Compared with the prior art, the present disclosure has the following advantages: the 250-bp cHS4 core region is truncated, which modified the configuration of the original core region; compared with existing 650-bp cHS4 insulator sequence in the prior art, the present disclosure not only significantly reduces the length of the insulator sequence, but also improves the protein expression amount; when used in protein transient expression, the present disclosure achieves an improved yield of transient protein expression, and meanwhile further reduces the production cost, hence possessing broad application prospects in the fields of antibody screening and production.

### BRIEF DESCRIPTION OF THE DRAWINGS

- FIG. 1: shows a vector map of P3-5ITR-3ITR-cHS4-TT.
- FIG. 2: shows a vector map of pcDNA3.4-PiggyBac.
- FIG. 3: shows the protein yield within 7 days from transient transfection.
- FIG. 4: shows the duration needed for plasmid construction and protein production.
- FIG. 5: shows the cost needed for transient transfection.
- FIG. 6: shows a vector map of P3-AscI.

### DETAILED DESCRIPTION

### Terminology

All publications, patents, and patent applications referred to in the specification are incorporated herein by reference to the same extent as if each individual publication, patent, or patent application was specifically and individually directed to be incorporated herein by reference.

Before the present disclosure is described in detail below, it will be appreciated that the present disclosure is not limited to the particular methodology, protocols, and reagents described herein, as these may vary. It will also be appreciated that the terminology used herein is only intended to describe specific embodiments rather than to limit the scope of the present disclosure. Unless otherwise defined, any technical and scientific term used herein has the same meaning as commonly understood by those of ordinary skill in the art to which the present disclosure belongs.

Certain embodiments disclosed herein encompass numerical ranges, and certain aspects of the present disclosure may be described by means of ranges. Unless otherwise indicated, it will be appreciated that the numerical ranges or the description by means of ranges are merely for the purposes of brevity and convenience, and should not be construed as a strict definition of the scope of the present disclosure. Accordingly, the description by means of ranges should be considered to have specifically disclosed all the possible subranges as well as all the possible specific numerical points within that range, as if those subranges and numerical points were explicitly disclosed herein. The principle described above applies regardless of the extent of the numerical values. When a range description is present, the range includes range endpoints.

When referring to a measurable value such as an amount, temporal duration of time, etc., the term "about" is meant to include a variation of ±20%, or ±10% in some cases, or ±5% in some cases, or ±1% in some cases, or ±0.1% in some cases, of the specified value.

As used herein, the term "antibody" typically refers to a Y-type tetrameric protein comprising two heavy (H) polypeptide chains and two light (L) polypeptide chains held together by covalent disulfide bonds and non-covalent interactions. Natural IgG antibodies have such a structure. Each light chain comprises a variable domain (VL) and a constant domain (CL). Each heavy chain comprises a variable domain (VH) and a constant domain (CH).

As used herein, the types of "antibodies" in a broad sense may include, e.g., polyclonal antibodies, monoclonal antibodies, chimeric antibodies, humanized and primatized antibodies, CDR-grafted antibodies, human antibodies (including human antibodies recombinantly produced), recombinantly produced antibodies, intracellular antibodies, multispecific antibodies, bifunctional fusion proteins, monovalent antibodies, multivalent antibodies, anti-idiotypic antibodies, synthetic antibodies (including mutant proteins and variants thereof), and the like.

The term "PiggyBac transposon expression system" refers to the PiggyBac transposon vector system. The PiggyBac vector system mainly comprises the following members: a helper vector or plasmid, which encodes PB (PiggyBac) transposase; and a transposon vector (also known as a donor vector) or plasmid, of which the two ends comprise optimized inverted terminal repeat (ITR) sequences; the region in between is a transposable region, into which a target gene sequence that is expected to be transposed into the host genome can be inserted. While carrying out the experiment, the target cell is co-transfected with the helper plasmid and the transposon plasmid, during which the transposase encoded by the helper plasmid recognizes and cleaves the ITR sequences at the two ends of the transposon plasmid. The released transposable region is integrated into a site containing a TTAA sequence in the host genome by the transposase, and TTAA repeats are present at both ends of the transposable region. Target cells may also be co-transfected with an *in vitro* transcribed transposase-encoding mRNA (in place of the helper plasmid) and the transposon plasmid for expression of the transposase.

The term "CHO platform" refers to a technical platform for screening CHO cell lines. Chinese hamster ovary cells (CHO) are acclimated in a culture medium with defined chemical ingredients, the CD CHO Fusion Medium, and subjected to subcloning and screening to establish a CHO cell line. The platform further includes supporting reagents and processes, such as an expression vector P3, a culture medium for the clone construction stage, a culture medium for a fed-batch process platform, and the like.

The term "vector" is meant to refer to a nucleic acid molecule capable of carrying another nucleic acid ligated to same, which may be single-stranded or double-stranded and includes DNA and RNA. One type of vector is a "plasmid", which refers to a circular double-stranded DNA loop into which an additional DNA segment can be ligated. Another type of vector is a viral vector, into which an additional DNA segment can be ligated into the viral genome. Certain vectors are capable of independent replication in a host cell into which they are introduced (e.g., bacterial vectors having a bacterial origin of replication and extrachromosomal mammalian vectors). Other vectors (e.g., non-extrachromosomal mammalian vectors) can be integrated into the genome of a host cell after introduction into the host cell, thereby being replicated along with the host genome.

The expression vector refers to a vector capable of expressing a target gene. The target gene includes, but is not limited to, a deoxynucleotide sequence encoding a product such as an antibody, an antigen, a fusion protein, a polypeptide, or the like, in a broad sense.

The term "insulator" or "insulator sequence" refers to a class of chromatin domain border DNA sequences that function as a neutral barrier to prevent the influence of the adjacent genetic elements or peripheral dense chromatin, so as to allow the normal spatiotemporal expression of protected genes. The effect of the insulator is related to the position where it is located in a gene and the direction of its own sequence. An exemplary insulator sequence includes chicken hypersensitive site-4 (cHS4).

The term "multiple cloning site" refers to an artificially synthesized DNA fragment contained in a vector; the multiple cloning site contains a plurality of single restriction enzyme cleavage sites that are insertion sites for exogenous DNAs and is also referred to as a multi-site linker. The multiple cloning site is a standard configuration sequence of a vector plasmid commonly used in genetic engineering. In the multiple cloning site, each restriction enzyme cleavage site is generally unique, that is, it is present only once in a particular vector plasmid. The enzyme cleavage sites of different enzymes may overlap.

The term "recombinant expression" refers to an oligonucleotide or polynucleotide construct comprising a genetic modification, and when the construct is contacted with a host cell under conditions sufficient to allow the expression of an mRNA, a protein, a polypeptide, or a peptide in the host cell, the construct allows the host cell to express the mRNA, the protein, the polypeptide, or the peptide; the construct comprises a nucleotide sequence encoding the mRNA, the protein, the polypeptide, or the peptide.

The term "transfection" refers to a specialized technique for introducing an exogenous gene into a cell, including chemical transfection, biological transfection, and physical transfection.

The term "transient expression" refers to the introduction of a constructed plasmid into a host cell by transfection, during which the exogenous gene in the plasmid is not integrated into the cell's own genome, but is present in the form of a plasmid. Along with cell growth and division, the exogenous gene will be gradually lost. During this period, the exogenous gene in the plasmid is transcribed and translated in the cell to express the protein.

### Examples

The present disclosure is further illustrated in detail by the following examples.

Specific examples are listed below to illustrate the present disclosure; however, it will be appreciated that these examples are listed only to illustrate the present disclosure rather than limit the scope of the present disclosure.

### Materials and reagents:

The optimization of the PB transposase-coding sequence for CHO cells (by adding an EcoRI enzyme cleavage site and a kozak sequence at the 5' end flank and adding HindIII at the 3' end flank) and its synthesis and cloning into the vector PUC57 to obtain PUC57-PiggyBac were accomplished by Synbio Technologies.

The template DNA sequence of the PiggyBac transposase-encoding mRNA was synthesized by Nanjing GenScript Biotech Co., Ltd.

The PiggyBac donor vector was synthesized and cyclized by Nanjing GenScript Biotech Co., Ltd.

The blank expression vector pcDNA3.4 was provided by Nanjing GenScript Biotech Co., Ltd.

### Example 1: Design and vector construction of truncated cHS4 insulator sequences

### 1. Design of truncated cHS4 insulator sequences

Among the used insulators, cHS4-650, cHS4-400, and cHS4-250 are derived from the literature (see, e.g., CN102943092A, WO2018083274A1, and US20150315611A1). The cHS4-650 sequence (including the 250-bp core region and the 400-bp sequence at the 3' end) was input into the AnimalTFDB3.0 online prediction website to predict transcription factors that may bind to it. The results showed a total of 3897 TFBSs, corresponding to 389 transcription factors. Then enrichment analysis was performed on the 389 transcription factors using the STRING database, and a total of 1993 GO terms were significantly enriched. The PPI analysis was performed on 91 transcription factors corresponding to 85 selected GO terms associated with chromatin structure regulation or insulator functionality. Then transcription factors with high connectivity and the 3 transcription factors, CTCF, USF1/2, and VEZF1, that are reported to bind to the core region were selected and positioned into the cHS4-650 sequence, according to the binding sequence predicted by AnimalTFDB3.0. DNA regions with less or no binding to the transcription factors were selected for truncation design, and the truncation was performed on a single region or a combination of multiple regions that can be truncated. Finally, 4 insulator sequences, SEQ ID NO. 1, SEQ ID NO. 2, SEQ ID NO. 3, and SEQ ID NO. 4, were obtained. Their nucleic acid sequences are shown below:
SEQ ID NO. 1 
cggggacagcccccccccaaagcccccagggatgtaattacgtccctcccccgctagggggcagcagcgagccgcccggggctccgctccg
gtccggcgctccccccgcatccccgagccggcagcgtgcggggacagcccgggcacggggaaggtggcacgggatcgctttcctctgaacgcttctc
gctgctctttgagcctgcagacacctggggggatacggggaaaatgtgtctgagcctgcatgtttgatggtgtctggatgcaagcagaaggggtggaaga
gcttgcctggagagatacagctgggtcagtaggactgggacaggcagctatccccaagaccaaccccaacccacccaccgtgcccactggccatgtcc
ttcatcacctccagggacggtgacccccccacctccgtgggcagctgtgccactgcagcaccgctctttggagaaggtaaatcttgctaaatccagcccg
 accctcccctggcacaacgtaaggccattatctct
SEQ ID NO. 2 
cggggacagcccccccccaaagcccccagggatgtaattacgtccctcccccgctagggggcagcagcgagccgcccggggctccgctccg
gtccggcgctccccccgcatccccgagccggcagcgtgcggggacagcccgggcacggggaaggtggcacgggatcgagcctgcagacacctgg
ggggatacggggaaaatgtgtctgagcctgcatgtttgatggtgtctggatgcaagcagaaggggtggaagagcttgcctggagagatacagctgggtc
agtaggactgggacaggcagctatccccaagaccaaccccaacccacccaccgtgcccactggccatgtccttcatcacctccagggacggtgacccc
cccacctccgtgggcagctgtgccactgcagcaccgctctttggagaaggtaaatcttgctaaatccagcccgaccctcccctggcacaacgtaaggcca
ttatctct
SEQ ID NO. 3 
cggggacagcccccccccaaagcccccagggatgtaattacgtccctcccccgctagggggcagcagcgagccgcccggggctccgctccg
gtccggcgctccccccgcatccccgagccggcagcgtgcggggacagcccgggcacggggaaggtggcacgggatcgctttcctctgaacgcttctc
gctgctctttgagcctgcagacacctggggggatgatgcaagcagaaggggtggaagagcttgcctggagagatacagctgggtcagtaggactggga
caggcagctatccccaagaccaaccccaacccacccaccgtgcccactggccatgtccttcatcacctccagggacggtgacccccccacctccgtgg
gcagctgtgccactgcagcaccgctctttggagaaggtaaatcttgctaaatccagcccgaccctcccctggcacaacgtaaggccattatctct
 SEQ ID NO. 4

cggggacagcccccccccaaagcccccagggatgtaattacgtccctcccccgctagggggcagcagcgagccgcccggggctccgctccg
gtccggcgctccccccgcatccccgagccggcagcgtgcggggacagcccgggcacggggaaggtggcacgggatcgagcctgcagacacctgg
ggggatgatgcaagcagaaggggtggaagagcttgcctggagagatacagctgggtcagtaggactgggacaggcagctatccccaagaccaaccc
caacccacccaccgtgcccactggccatgtccttcatcacctccagggacggtgacccccccacctccgtgggcagctgtgccactgcagcaccgctctt
tggagaaggtaaatcttgctaaatccagcccgaccctcccctggcacaacgtaaggccattatctct

### 2. Donor vector construction

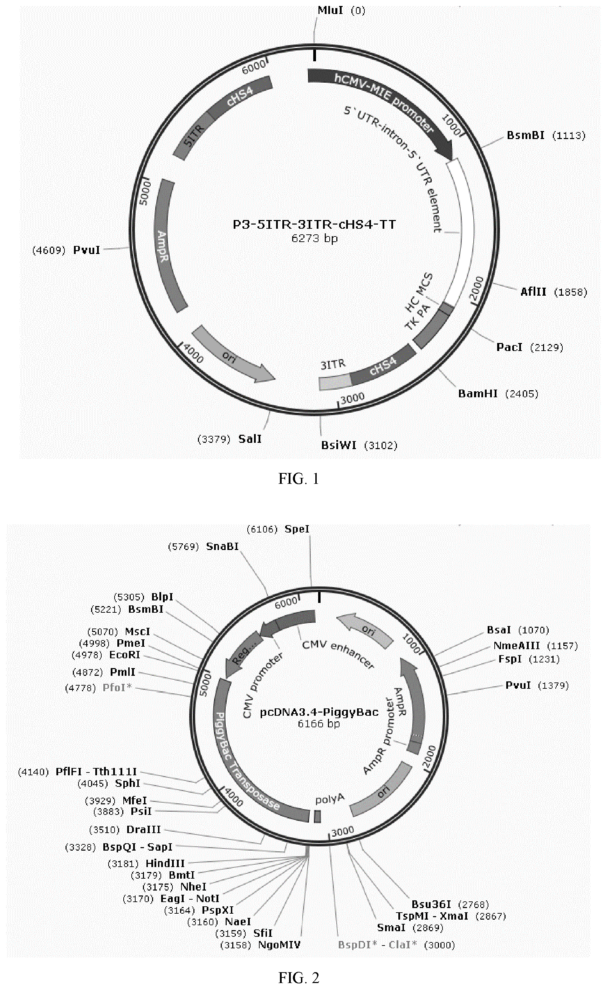

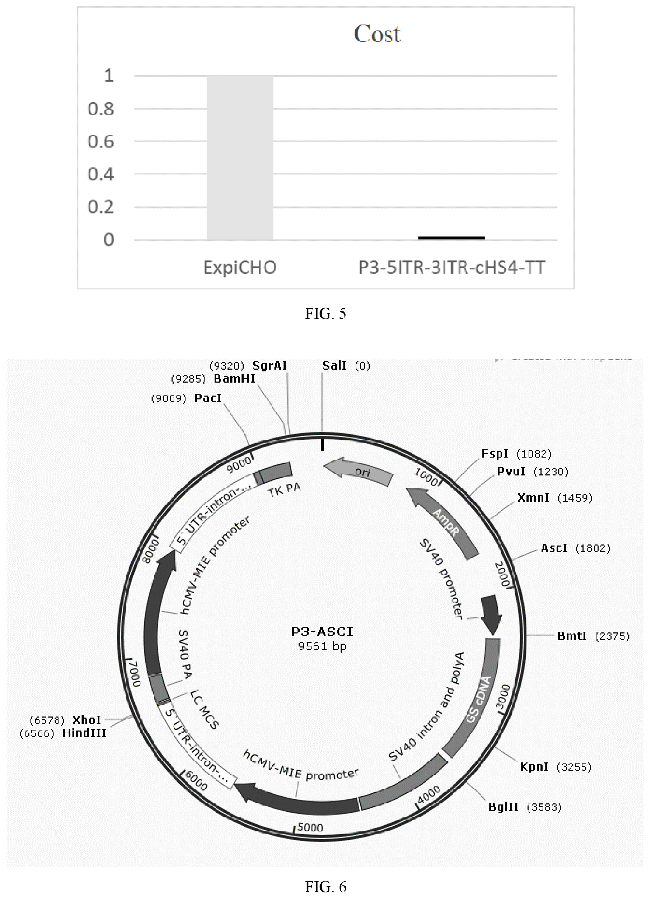

A donor vector was constructed using SEQ ID NO. 1 as an exemplary truncated insulator. The PiggyBac donor vector element contains 5ITR-cHS4 (forward) and 3ITR-cHS4 (forward), where there are two forward cHS4 between 5ITR and 3ITR, and cHS4 comprises the nucleic acid sequence shown in SEQ ID NO. 1. On the basis of a P3-AscI vector (as shown in FIG. 6, constructed by inserting an AscI enzyme cleavage site recognition sequence GGCGCGCC into a P3 plasmid), the screening marker GS gene expression cassette and one target gene expression cassette were deleted; elements 5ITR and cHS4 were added by means of AscI single enzyme cleavage and ligation or homologous recombination, and elements 3ITR and cHS4 were added by means of SgrAI single enzyme cleavage and ligation or homologous recombination to obtain the PiggyBac donor vector sequence, which was named P3-5ITR-3ITR-cHS4-TT (FIG. 1).

### 3. Expression vector construction

The vector P3-5ITR-3ITR-cHS4-TT was subjected to dual enzyme cleavage and ligation using BstBI/PacI, and the S1 chain- and the S2 chain-coding nucleic acid sequences of the QL01 protein (preserved by the applicant) are separately ligated in the multiple cloning site to construct recombinant expression vectors P3-5ITR-3ITR-cHS4-TT-QL01-S1 and P3-5ITR-3ITR-cHS4-TT-QL02-S2. The coding nucleic acid sequence of the QL02 protein (preserved by the applicant) was ligated to the multiple cloning site to construct a recombinant expression vector P3-5ITR-3ITR-cHS4-TT-QL02.

### 4. Helper vector construction

The vector PUC57-PiggyBac and the blank vector pcDNA3.4 were subjected to dual enzyme cleavage using EcoRI/HindIII, and the enzymatically cleaved products were purified and recovered by the NucleoSpin Gel and PCR Clean-up kit and then ligated to construct a helper plasmid pcDNA3.4-PiggyBac containing the PB transposase-encoding gene (FIG. 2). Also, the PB transposase-encoding mRNA-PiggyBac may be obtained by *in vitro* transcription, capping, and tailing modifications using a PB transposase-encoding DNA sequence as a template by means of the *in vitro* transcription method.

### Example 2: Antibody expression by transient transfection with truncated cHS4 insulator sequences

Transient transfection expression of the expression vector containing the truncated cHS4 insulator sequences: The used host cell was CHOZN (from Merck). The CHO cells were transfected with plasmid amounts of 60 µg of the expression vector + 6 µg of the helper vector, by the electrotransfection method at a voltage of 300 V, 950 µF, and a cell amount of 1E7 for electric shock once. After the cell transfection, the cells were resuspended in the culture medium EX-CELL^{®} CD CHO Fusion Medium (containing 6 mM L-glutamine) and placed in an incubator for static culture under the condition of 37°C with 5% CO₂. After 24 h of culture, the cells were counted, and the viable cell density (VCD) and the cell viability (Via) were determined. Then the cells were centrifuged, resuspended in an appropriate amount of a culture medium at a density of 6 × 106 cells/mL, and subjected to 7 days of fed-batch protein expression production.

As a control group, the ExpiCHO^{™} kit was used to perform transient expression of the protein according to the recommended method: The expression system was 25 mL, and the total amount of plasmids for transfection was 20 µg. The transfection and feed addition were performed according to the recommendations for the kit, and the culture was conducted for 7 days.

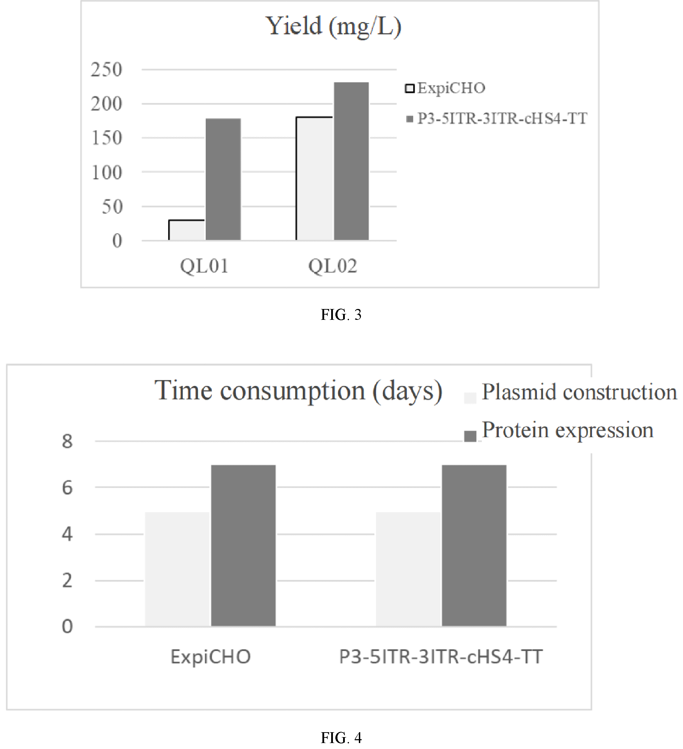

All the antibody expression amounts were measured on the Cedex system, and the test results are shown in FIG. 3.

By comparing the transient transfection results of the two systems, the protein yields of the P3-5ITR-3ITR-cHS4-TT expression system were increased by 488.2% (QL01) and 29.6% (QL02), as compared with ExpiCHO^{™}. The expression system of the present disclosure also achieved a relatively good expression effect for protein QL01 that has relatively high production difficulty in existing expression systems, and the durations for plasmid construction and for protein expression production of the two systems were comparable (see FIG. 4), while the cost of the present disclosure was reduced by 96% or more (see FIG. 5).

Therefore, the present disclosure provides a transient expression system with a simple process, low industrial costs, and high protein yield.

The embodiments of the present disclosure described above are merely exemplary and shall not be construed as limitations on the embodiments of the present disclosure. Any of those skilled in the art can recognize or determine numerous equivalents of specific compounds, materials, and operations without the need for undue experimentation. All such equivalents shall fall within the scope of the present disclosure, and are encompassed by the claims.

## Claims

1. An insulator, comprising the nucleic acid sequence shown in SEQ ID NO: 1, SEQ ID NO: 2, SEQ ID NO: 3, or SEQ ID NO: 4, or comprising a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to any one of the nucleic acid sequences shown in SEQ ID NO: 1, SEQ ID NO: 2, SEQ ID NO: 3, and SEQ ID NO: 4.

2. The insulator according to claim 1, wherein the insulator is the nucleic acid sequence shown in SEQ ID NO: 1, SEQ ID NO: 2, SEQ ID NO: 3, or SEQ ID NO: 4, or is a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to any one of the nucleic acid sequences shown in SEQ ID NO: 1, SEQ ID NO: 2, SEQ ID NO: 3, and SEQ ID NO: 4.

3. An expression vector, comprising the insulator according to claim 1 or 2.

4. The expression vector according to claim 3, wherein the expression vector is a PiggyBac transposon transgenic vector; preferably, the expression vector does not comprise a marker gene for screening, or preferably, the expression vector comprises a marker gene for screening.

5. An expression system, comprising the insulator according to claim 1 or 2.

6. The expression system according to claim 5, wherein the expression system is a PiggyBac transposon expression system.

7. The expression system according to claim 6, wherein the PiggyBac transposon expression system comprises a PiggyBac transposon transgenic vector; preferably, the PiggyBac transposon transgenic vector comprises the insulator according to claim 1 or 2; preferably, the PiggyBac transposon transgenic vector comprises a marker gene for screening, or preferably, the PiggyBac transposon transgenic vector does not comprise a marker gene for screening.

8. The expression vector according to claim 4 or the expression system according to claim 7, wherein the PiggyBac transposon transgenic vector comprises a 5' terminal repeat 5ITR and an inverted 3' terminal repeat 3ITR, and the insulator is arranged between the 5ITR and the 3ITR; preferably, two insulators that are inverted repeats or direct repeats are sequentially arranged between the 5ITR and the 3ITR; preferably, a multiple cloning site is arranged between the insulators; preferably, the other elements of the PiggyBac transposon transgenic vector are derived from an original sequence of a P3 vector.

9. The expression system according to any one of claims 6-8, wherein the PiggyBac transposon expression system comprises a PiggyBac helper vector or an mRNA encoding a PB transposase, and the PiggyBac helper vector comprises a nucleic acid encoding a PB transposase.

10. The expression system according to claim 9, wherein the PiggyBac helper vector is constructed from pcDNA3.4.

11. A method for producing a protein by transient expression, comprising the following steps:
1) constructing an expression system comprising a gene encoding a target protein;
2) introducing the expression system in step 1) into a host cell by transfection; and
3) using a pool of the transfected cells for expression production after the transfection;
wherein the expression system comprises the insulator according to claim 1 or 2.

12. The method according to claim 11, wherein the target protein is an antibody, a fusion protein, an antigen, an enzyme, or a protein or polypeptide of other types.

13. The method according to claim 11 or 12, wherein a method for the transfection is electrotransfection, PEI or liposome transfection, or other transfection methods.

14. The method according to any one of claims 11-13, wherein the method does not comprise a stressed screening procedure.

15. The method according to any one of claims 11-14, wherein the host cell is a mammalian cell, preferably a CHO cell.

16. The method according to any one of claims 11-15, wherein the expression system is selected from the expression system according to any one of claims 5-10.

17. Use of the insulator according to claim 1 or 2, the expression vector according to any one of claims 3-4 and 8, or the expression system according to any one of claims 5-10 in the recombinant expression of a nucleic acid, wherein preferably, the recombinant expression does not comprise a stressed screening procedure.

18. The use according to claim 17, wherein the nucleic acid encodes an antibody, a fusion protein, an antigen, an enzyme, or a protein or polypeptide of other types.

19. Use of the insulator according to claim 1 or 2, the expression vector according to any one of claims 3-4 and 8, or the expression system according to any one of claims 5-10 in preparing a protein or polypeptide.

20. The use according to claim 19, wherein the protein or polypeptide comprises an antibody, a fusion protein, an antigen, and an enzyme.

21. Use of the insulator according to claim 1 or 2, the expression vector according to any one of claims 3-4 and 8, or the expression system according to any one of claims 5-10 in constructing a recombinant cell line.

22. The use according to claim 21, wherein the recombinant cell is a mammalian cell, preferably a CHO cell.
